(19) Europäisches Patentamt

European Patent Office

Office européen des brevets

(11) **EP 0 706 787 B1**

(12) **EUROPÄISCHE PATENTSCHRIFT**

(45) Veröffentlichungstag und Bekanntmachung des Hinweises auf die Patenterteilung:
**16.02.2000 Patentblatt 2000/07**

(51) Int. Cl.⁷: **A61K 7/13**

(21) Anmeldenummer: 95109087.7

(22) Anmeldetag: **13.06.1995**

(54) **Oxidationshaarfärbemittel**

Oxidation hair dye

Colorant d'oxydation pour cheveux

(84) Benannte Vertragsstaaten:
**DE ES FR GB IT**

(30) Priorität: **14.10.1994 DE 4436743**

(43) Veröffentlichungstag der Anmeldung:
**17.04.1996 Patentblatt 1996/16**

(73) Patentinhaber:
**Wella Aktiengesellschaft**
**64274 Darmstadt (DE)**

(72) Erfinder: **Mager, Herbert, Dr.**
**CH-1723 Marly (CH)**

(56) Entgegenhaltungen:
| | |
|---|---|
| **EP-A- 0 376 047** | **EP-A- 0 400 330** |
| **EP-A- 0 624 362** | **EP-A- 0 634 162** |
| **EP-A- 0 634 163** | **EP-A- 0 634 164** |
| **EP-A- 0 634 165** | **DE-A- 3 834 142** |
| **DE-A- 3 942 294** | **DE-A- 4 017 516** |
| **FR-A- 2 706 297** | |

**Beschreibung**

Gegenstand der Erfindung ist ein Mittel zum oxidativen Färben von Haaren auf der Basis eines rotfärbenden Nitrofarbstoffes und einer Entwicklersubstanz/Kupplersubstanz-Kombination.

In der Haarfärbepraxis haben Oxidationshaarfärbemittel eine wesentliche Bedeutung erlangt. Die Färbung erfolgt hierbei im Haarschaff durch die Reaktion bestimmter Entwicklersubstanzen mit bestimmten Kupplersubstanzen in Gegenwart eines geeigneten Oxidationsmittels. Zusätzlich zu den Entwicklersubstanzen und Kupplersubstanzen können Oxidationshaarfärbemittel direktziehende Farbstoffe zur Nuancierung enthalten. Derartige Farbstoffkombinationen enthaltende spezielle cremeförmige Farbträgermassen werden beispielsweise in der DE-OS 38 34 142 beschrieben.

An Oxidationsfarbstoffe und direktziehende Farbstoffe, die zur Färbung menschlicher Haare verwendet werden, sind zahlreiche besondere Anforderungen gestellt. So müssen sie in toxikologischer und dermatologischer Hinsicht unbedenklich sein und Färbungen mit der gewünschten Intensität ermöglichen. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens vier bis sechs Wochen stabil bleiben.

Zur Erzielung modischer Nuancen ist vor allem der aus der EP-OS 0 376 047 bekannte direktziehende Nitrofarbstoff 2-Chlor-6-ethylamino-4-nitrophenol geeignet.

Bei alleiniger Verwendung von 2-Chlor-6-ethylamino-4-nitrophenol wird in Gegenwart eines Oxidationsmittels ein neutrales Rot erzielt. Die Farbintensität dieser Rotfärbung ist jedoch begrenzt und kann durch eine Erhöhung der Konzentration des Farbstoffes nur in geringem Umfang verstärkt werden. Eine Konzentrationserhöhung ist zudem wegen der begrenzten Löslichkeit des Farbstoffes in dem verwendeten Haarfärbemittel nur bedingt und nicht immer im notwendigen Umfang möglich.

Es hat sich weiterhin gezeigt, daß bei Verwendung von 2-Chlor-6-ethylamino-4-nitrophenol zur Färbung von längerem oder geschädigtem Haar der für eine gleichmäßige Haarfärbung erforderliche Farbausgleich nicht immer in dem erforderlichem Umfang erfolgt und in dieser Hinsicht Verbesserungen des Färbeergebnisses notwendig sind.

Es wurde nunmehr Überraschenderweise gefunden, daß die geschilderten Nachteile vermieden werden können, wenn 2-Chlor-6-ethylamino-4-nitrophenol gemeinsam mit der Kupplersubstanz 5-Amino-2-methylphenol und der Entwicklersubstanz 1,4-Diaminobenzol und/oder 2,5-Diaminotoluol und/oder 2,5 Diaminophenylethanol in Oxidationshaarfärbemitteln verwendet wird.

Gegenstand der vorliegenden Erfindung ist daher ein Mittel zur oxidativen Färbung von Haaren enthaltend einen direktziehenden Farbstoff und eine Entwicklersubstanz/Kupplersubstanz-Kombination, dadurch gekennzeichnet, daß es als direktziehenden Farbstoff 2-Chlor-6-ethylamino-4-nitrophenol und als Kupplersubstanz 5-Amino-2-methylphenol enthält, und die Entwicklersubstanz ausgewählt ist aus 1,4-Diaminobenzol, 2,5-Diaminotoluol und 2,5-Diaminophenylethanol oder deren Gemischen.

Das mit dem erfindungsgemäßen Oxidationshaarfärbemittel behandelte Haar ist gleichmäßig und intensiv rot bis rotviolett gefärbt.

In dem Haarfärbemittel soll das 2-Chlor-6-ethylamino-4-nitrophenol in einer Menge von 0,01 bis 2 Gewichtsprozent enthalten sein, während die Einsatzmenge der Kupplersubstanz 5-Amino-2-methylphenol 0,001 bis 3 Gewichtsprozent betragen soll.

Die Entwicklersubstanzen 1,4-Diaminobenzol, 2,5-Diaminotoluol und 2,5-Diaminophenylethanol können sowohl alleine als auch im Gemisch miteinander eingesetzt werden, wobei die Gesamteinsatzmenge dieser Entwicklersubstanzen 0,001 bis 4 Gewichtsprozent betragen soll. Obwohl die vorteilhaften Eigenschatten der vorstehenden Farbstoffkombination es nahegelegen, diese ohne den Zusatz von weiteren Farbstoffen zu verwenden, ist es selbstverständlich auch möglich, neben der Kupplersubstanz 5-Amino-2-methylphenol zusätzlich weitere übliche Kupplersubstanzen, wie zum Beispiel 4-Hydroxyindol, 2-Methylresorcin, 1-Naphthol, 3-Aminophenol, 3,5-Diamino-2,6-dimethoxypyridin einzusetzen.

Weiterhin können neben den Entwicklersubstanzen 1,4-Diaminobenzol, 2,5-Diaminotoluol und 2,5-Diaminophenylethanol weitere übliche Entwicklersubstanzen, beispielsweise 4-Amino-3-methylphenol, enthalten sein. Derartige zur Haarfärbung bekannte und übliche Entwicklersubstanzen sind unter anderem in dem Buch von E. Sagarin "Cosmetics, Science and Technology" (1957), Interscience Publishers Inc., New York, Seiten 503 ff. sowie in dem Buch von H. Janistyn, "Handbuch der Kosmetika und Riechstoffe" (1973), Band 3, Seiten 388 ff. beschrieben.

Diese Kupplersubstanzen und Entwicklersubstanzen können in dem erfindungsgemäßen Haarfärbemittel jeweils einzeln oder im Gemisch miteinander enthalten sein.

Die Gesamtmenge der in dem hier beschriebenen Haarfärbemittel enthaltenen Entwicklersubstanz-Kupplersubstanz-Kombination beträgt 0,1 bis 5,0 Gewichtsprozent, wobei eine Menge von 0,5 bis 4,0 Gewichtsprozent bevorzugt ist. Die Entwicklerkomponente wird im allgemeinen in einer etwa äquimolaren Menge, bezogen auf die Kupplerkomponente, eingesetzt. Es ist jedoch nicht nachteilig, wenn die Entwicklerkomponente diesbezüglich in einem gewissen Überschuß oder Unterschuß vorhanden ist.

Weiterhin kann das erfindungsgemäße Haarfärbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie Basic Violet 14 (C.I. 42 510) und Basic Violet 2 (C.I. 42 520), aromatische Nitrofarbstoffe wie 2-Amino-4-nitrophenol, 2-Amino-5-nitrophenol, 2-Amino-4,6-dinitrophenol, 2-Amino-5-(2'-hydroxyethyl)amino-nitrobenzol und 2-Methylamino-5-bis-(2'-hydroxyethyl)amino-nitrobenzol, Azofarbstoffe wie Acid Brown 4 (C.I. 14 805) und Dispersionsfarbstoffe wie beispielsweise 1,4-Diaminoanthrachinon und 1,4,5,8-Tetraaminoanthrachinon enthalten. Die Haarfärbemittel können diese Farbkomponenten in einer Menge von etwa 0,1 bis 4,0 Gewichtsprozent enthalten.

Selbstverständlich können die Kuppler- und Entwicklersubstanzen sowie die anderen Farbkomponenten - sofern es Basen sind - auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in dem Haarfärbemittel noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des neuen Haarfärbemittels kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion.

Ihre Zusammensetzung stellt eine Mischung der Farbstoffkomponenten mit den für solchen Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol, Isopropanol und Glycerin, oder Glykole, beispielsweise 1,2-Propylenglykol, weiterhin Netzmittel oder Emulgatoren aus den Klassen der anionischen, kationischen, amphoteren oder nichtionogenen oberflächenaktiven Substanzen, beispielsweise Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke, Cellulosederivate, Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, neutral oder alkalisch reagieren. Insbesondere weist es einen pH-Wert von 6,0 bis 11,5 auf, wobei die Einstellung vorzugsweise mit Ammoniak erfolgt. Es können aber auch organische Amine, zum Beispiel Monoethanolamin und Triethanolamin, oder auch organische Basen wie Natriumhydroxid und Kaliumhydroxid Verwendung finden. Für eine pH-Einstellung im sauren Bereich kommen zum Beispiel Phosphorsäure, Essigsäure, Zitronensäure oder Weinsäure in Betracht.

Für die Anwendung zur oxidativen Färbung von Haaren vermischt man das vorstehend beschriebene Haarfärbemittel üblicherweise unmittelbar vor dem Gebrauch mit einem Oxidationsmittel und trägt eine für die Haarfärbebehandlung ausreichende Menge, je nach Haarfülle im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form einer 3- bis 12-prozentigen, vorzugsweise einer 6-prozentigen, wäßrigen Lösung aber auch Luftsauerstoff in Betracht. Wird eine 6-prozentige Wasserstoffperoxidlösung als Oxidationsmittel verwendet, so beträgt das Gewichtsverhältnis vorzugsweise 5 : 1 bis 1 : 3, wobei ein Verhältnis von 1 : 1 bis 1 : 2 besonders bevorzugt ist. Größere Mengen an Oxidationsmittel werden vor allem bei höheren Farbstoffkonzentrationen im Haarfärbemittel oder wenn gleichzeitig eine stärkere Bleichung der Haare beabsichtigt ist, verwendet.

Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die nachfolgenden Beispiele sollen den Gegenstand der Erfindung näher erläutern ohne diesen hierauf zu beschränken.

Beispiele

Beispiel 1: Haarfärbecreme

| | |
|---|---|
| 0,80 g | 2-Chlor-6-ethylamino-4-nitrophenol |
| 0,10 g | 2,5-Diaminotoluolsulfat |
| 0,06 g | 5-Amino-2-methylphenol |
| 15,00 g | Cetylalkohol |
| 6,50 g | Ammoniak (25-prozentige wäßrige Lösung) |
| 3,50 g | Laurylalkoholdiglykolethersulfat) (28-prozentige wäßrige Lösung) |
| 74,04 g | Wasser |
| 100,00 g | |

20 g der obigen Haarfärbecreme werden mit 40 g einer 6-prozentigen Wasserstoffperoxidlösung vermischt. Anschließend wird das so erhaltene gebrauchsfertige Haarfärbemittel auf gebleichtes Haar aufgetragen und 30 Minuten lang bei Raumtemperatur (20 bis 30 Grad Celsius) einwirken gelassen. Sodann wird das Haar mit Wasser ausgespült und getrocknet. Es wird eine farbsatte, leuchtend rote Haarfärbung erhalten.

Beispiel 2: Haarfärbegel

| | |
|---|---|
| 0,60 g | 2-Chlor-6-ethylamino-4-nitrophenol |
| 0,15 g | 2,5-Diaminophenylethanolsulfat |
| 0,10 g | 5-Amino-2-methylphenol |
| 0,05 g | Resorcin |
| 15,00 g | Ölsäure |
| 10,00 g | Ammoniak (25-prozentige wäßrige Lösung) |
| 7,00 g | Isopropanol |
| 0,30 g | Ascorbinsäure |
| 66,80 g | Wasser |
| 100,00 g | |

Unmittelbar vor Gebrauch werden 50 g des obigen Haarfärbegels mit 50 g einer 6-prozentigen Wasserstoffperoxidlösung vermischt. Anschließend wird das erhaltene Haarfärbemittel auf hellblonde menschliche Haare aufgetragen und 30 Minuten lang bei 40 Grad Celsius einwirken gelassen. Sodann wird das Haar mit Wasser gespült und getrocknet.

Das so behandelte Haar weist einen gleichmäßigen und intensiven Mahagoniton auf.

4

Beispiel 3: Vergleichsversuche

Beispiel 3A

Eine Färbecreme gemäß Beispiel 1 wird mit der folgenden nicht-erfindungsgemäßen Färbecreme (Beispiel 1a) verglichen:

Beispiel 1a: Färbecreme

| | |
|---|---|
| 0,80 g | 2-Chlor-6-ethylamino-4-nitrophenol |
| 15,00 g | Cetylalkohol |
| 6,50 g | Ammoniak (25-prozentige wäßrige Lösung) |
| 3,50 g | Laurylalkoholdiglykolethersulfat (28-prozentige wäßrige Lösung) |
| 0,30 g | Natriumsulfit, wasserfrei |
| 73,90 g | Wasser |
| 100,00 g | |

20 g der Färbecreme gemäß Beispiel 1 bzw. Beispiel 1a werden mit 40 g einer 6-prozentigen Wasserstoffperoxidlösung vermischt. Sodann werden die erhaltenen Haarfärbemittel jeweils auf handelsübliche gebleichte Büffelhaarsträhnen aufgetragen. Nach einer Einwirkungszeit von 30 Minuten bei Raumtemperatur werden die Büffelhaarsträhnen mit Wasser ausgespült und getrocknet.

Die mit dem erfindungsgemäßen Haarfärbemittel (Beispiel 1) erzielte Haarfärbung ist deutlich farbsatter als die mit dem nicht-erfindungsgemäßen Haarfärbemittel (Beispiel 1a) erhaltene Haarfärbung, was auch durch den Vergleich des mit einem Minolta Chromameter,Typ II ermittelten Helligkeitswertes L eindeutig belegt wird.

Helligkeitswert L (Beispiel 1): 33,3
Helligkeitswert L (Beispiel 1a): 40,0.

Hierbei bedeutet ein geringerer Helligkeitswert eine höhere Farbsättigung.

Beispiel 3B:

Die Vergleichsversuche gemäß Beispiel 3A wurden wiederholt, wobei jedoch die Büffelhaarsträhnen vor der Färbung mit einem handelsüblichen Bleichmittel oxidativ geschädigt wurden (Einwirkungszeit des Bleichmittels: 60 Minuten bei 40 Grad Celsius).

Die ermittelten Helligkeitswerte belegen eindeutig, daß auch in diesem Fall mit dem erfindungsgemäßen Haarfärbemittel eine gegenüber dem nicht-erfindungsgemäßen Haarfärbemittel wesentlich bessere Färbung erzielt wird.

Helligkeitswert L' (Beispiel 1): 36,7
Helligkeitswert L' (Beispiel 1a): 50,2.

Zusätzlich wurde die Kennzahl für den erzielten Farbausgleich auf unterschiedlich geschädigtem Haar ermittelt. Hierzu wurden jeweils die Helligkeitswerte der Färbung, welche mit vorheriger Haarbleichung erzielt wurde, durch die Helligkeitswerte der Färbung, welche ohne vorherige Haarbleichung erzielt wurde, dividiert.

## EP 0 706 787 B1

Kennzahl für den Farbausgleich des erfindungsgemäßen Haarfärbemittels (Beispiel 1)

L' : L = 36,7 : 33,3 = 1,10

Kennzahl für den Farbausgleich des nicht-erfindungsgemäßen Haarfärbemittels (Beispiel 1a)

L' : L = 50,2 : 40,0 = 1,26

Die Kennzahl beträgt im Idealfall 1 (gleiche Färbung bei geschädigtem und ungeschädigtem Haar), so daß das Farbausgleichvermögen umso besser ist, je näher die Kennzahl bei 1 liegt.

Der Vergleich der Kennzahlen für das erfindungsgemäße und das nicht-erfindungsgemäße Haarfärbemittel zeigt deutlich, daß das erfindungsgemäße Haarfärbemittel weniger empfindlich auf eine unterschiedliche Haarschädigung reagiert als das nicht-erfindungsgemäße Haarfärbemittel und daher einen besseren Farbausgleich und eine gleichmäßigere Haarfärbung ermöglicht.

Alle Prozentangaben stellen, soweit nicht anderes angegeben ist, Gewichtsprozent dar.

**Patentansprüche**

1. Mittel zur oxidativen Färbung von Haaren, enthaltend einen direktziehenden Farbstoff und eine Entwicklersubstanz/Kupplersubstanz-Kombination, dadurch gekennzeichnet, daß es als direktziehenden Farbstoff 2-Chlor-6-ethylamino-4-nitrophenol und als Kupplersubstanz 5-Amino-2-methylphenol enthält, und die Entwicklersubstanz ausgewählt ist aus 1,4-Diaminobenzol, 2,5-Diaminotoluol und 2,5-Diaminophenylethanol oder deren Gemischen.

2. Mittel nach Anspruch 1, dadurch gekennzeichnet, daß das 2-Chlor-6-ethylamino-4-nitrophenol in einer Menge von 0,01 bis 2 Gewichtsprozent enthalten ist.

3. Mittel nach Anspruch 1 oder 2, dadurch gekennzeichnet, daß das 5-Amino-2-methylphenol in einer Menge von 0,001 bis 3 Gewichtsprozent enthalten ist.

4. Mittel nach einem der Ansprüche 1 bis 3, dadurch gekennzeichnet, daß die Entwicklersubstanz in einer Menge von 0,001 bis 4 Gewichtsprozent enthalten ist.

5. Mittel nach einem der Ansprüche 1 bis 4, dadurch gekennzeichnet, daß die Entwicklersubstanz/Kupplersubstanz-Kombination in einer Menge von 0,1 bis 5 Gewichtsprozent enthalten ist.

6. Mittel nach einem der Ansprüche 1 bis 5, dadurch gekennzeichnet, daß es als Antioxidantien Natriumsulfit, Ascorbinsäure oder Thioglykolsäure enthält.

7. Verfahren zur oxidativen Färbung von Haaren, dadurch gekennzeichnet, daß man unmittelbar vor Gebrauch ein Haarfärbemittel nach einem der Ansprüche 1 bis 6 mit einem Oxidationsmittel vermischt, sodann eine für die Haarfärbung ausreichende Menge dieses Gemisches auf das Haar aufträgt, es dort 10 bis 45 Minuten bei 15 bis 50 Grad Celsius einwirken läßt, anschließend das Haar mit Wasser spült und sodann trocknet.

8. Verfahren nach Anspruch 7, dadurch gekennzeichnet, daß das Oxidationsmittel ausgewählt ist aus Wasserstoffperoxid und dessen Additionsverbindungen an Harnstoff, Melamin oder Natriumborat.

9. Verfahren nach Anspruch 7 oder 8, dadurch gekennzeichnet, daß als Oxidationsmittel eine 6-prozentige Wasserstoffperoxidlösung verwendet wird und das Haarfärbemittel mit dem Oxidationsmittel in einem Gewichtsverhältnis von 5 : 1 bis 1 : 3 vermischt wird.

**Claims**

1. Agent for the oxidative dyeing of hair, containing a direct dye and a developer substance/coupler substance combination, characterised in that it contains 2-chloro-6-ethylamino-4-nitrophenol as the direct dye, and 5-amino-2-methylphenol as the coupler substance, and the developer substance is selected from the group consisting of 1,4-diaminobenzene, 2,5-diaminotoluene and 2,5-diaminophenylethanol or mixtures thereof.

2. Agent according to Claim 1, characterised in that it contains from 0.01 to 2 % by weight of 2-chloro-6-ethylamino-4-nitrophenol.

3. Agent according to Claim 1 or 2, characterised in that it contains from 0.001 to 3 % by weight of 5-amino-2-methylphenol.

4. Agent according to any one of Claims 1 to 3, characterised in that it contains from 0.001 to 4 % by weight of developer substance.

5. Agent according to any one of Claims 1 to 4, characterised in that it contains from 0.1 to 5 % by weight of developer substance/coupler substance combination.

6. Agent according to any one of Claims 1 to 5, characterised in that it contains sodium sulphite, ascorbic acid or thioglycolic acid as anti-oxidant.

7. Process for the oxidative dyeing of hair, characterised in that, immediately before use, a hair dyeing agent according to any one of Claims 1 to 6 is mixed with an oxidising agent, then an amount of that mixture sufficient to dye the hair is applied to the hair and is left there to take effect for from 10 to 45 minutes at from 15 to 50°C, and the hair is subsequently rinsed with water and then dried.

8. Process according to Claim 7, characterised in that the oxidising agent is selected from hydrogen peroxide and its addition compounds with urea, melamine or sodium borate.

9. Process according to Claim 7 or 8, characterised in that a 6 % hydrogen peroxide solution is used as the oxidising agent and the hair dyeing agent is mixed with the oxidising agent in a ratio by weight of from 5:1 to 1:3.

**Revendications**

1. Agent de coloration oxydative des cheveux, contenant un colorant à application directe et une combinaison substance révélateur/substance de couplage, caractérisé en ce qu'il contient à titre de colorant à application directe du 2-chloro-6-éthylamino-4-nitrophénol et comme substance de couplage du 5-amino-2-méthylphénol et la substance révélatrice est choisie dans le groupe constitué de 1,4-Diaminobenzène, 2,5-diaminotoluène et 2,5-Diaminophényléthanol ou leurs mélanges.

2. Agent selon la revendication 1, caractérisé en ce que le 2-chloro-6-éthylamino-4-nitrophénol est contenu en une quantité comprise en la plage allant de 0,01 à 2% en poids.

3. Agent selon l'une des revendications 1 ou 2, caractérisé en ce que le 5-amino-2-méthyiphénol est contenu en une quantité comprise dans la plage allant de 0,001 à 3 % en poids.

4. Agent selon l'une des revendications 1 à 3, caractérisé en ce que la substance révélatrice est contenue en une quantité comprise dans la plage allant de 0,001 à 4 % en poids.

5. Agent selon l'une des revendications 1 à 4, caractérisé en ce que la combinaison substance révélatrice/substance de couplage est contenu en une quantité comprise entire 0,1 et 5 % en poids.

6. Agent selon l'une des revendications 1 à 5, caractérisé en ce qu'il contient comme antioxydant du sulfite de sodium, de l'acide ascorbique ou de l'acide thioglycolique.

7. Procédé de coloration oxydative des cheveux, caractérisé an ce que l'on mélange directement avant utilisation un agent de coloration des cheveux selon l'une des revendications 1 à 6 avec un agent oxydant, puis on applique sur les cheveux une quantité de ce mélange suffisante pour obtenir la coloration des chevaux, on l'y laisse agir de 10 à 45 minutes à une température de 15 à 50 °C, puis on rince les cheveux à l'eau et l'on sèche.

8. Procédé selon la revendication 7, caractérisé en ce que l'agent oxydant est choisi parmi le peroxyde d'hydrogène et ses combinaisons d'addition, faisant appel à l'urée, la mélamine et au borate de sodium.

9. Procédé selon la revendication 7 ou la revendication 8, caractérisé an ce qu'on utilise comme agent oxydant une solution de peroxyde d'hydrogène à 6 % et l'agent de coloration des cheveux est mélangé à l'agent oxydant en une proportion en poids comprise dans la plage allant de 5 : 1 à 1 : 3.